Europäisches Patentamt

European Patent Office

Office européen des brevets

(19)

(11) Veröffentlichungsnummer: **0 103 697**
**A1**

(12) **EUROPÄISCHE PATENTANMELDUNG**

(21) Anmeldenummer: **83107023.0**

(22) Anmeldetag: **18.07.83**

(51) Int. Cl.³: **A 61 F 1/03**

(30) Priorität: **22.09.82 CH 5594/82**

(43) Veröffentlichungstag der Anmeldung:
**28.03.84 Patentblatt 84/13**

(84) Benannte Vertragsstaaten:
**AT CH DE FR IT LI**

(71) Anmelder: **GEBRÜDER SULZER AKTIENGESELLSCHAFT**
**Zürcherstrasse 9**
**CH-8401 Winterthur(CH)**

(72) Erfinder: **Burch, Hansbeat, Dr.-med.**
**Kantonsspital**
**CH-1700 Freiburg(CH)**

(72) Erfinder: **Van der Roer, Humphrey A.V.**

**CH-1782 Belfaux(CH)**

(74) Vertreter: **Dipl.-Ing. H. Marsch Dipl.-Ing. K. Sparing**
**Dipl.-Phys.Dr. W.H. Röhl Patentanwälte**
**Rethelstrasse 123**
**D-4000 Düsseldorf(DE)**

(54) **Kniegelenkprothese.**

(57) Bei einer Beugung des Knies rollen die kondylenartigen Tragflächen (4) des Femurteils (5) auf den tibiaartigen Abrollflächen (3) des Tibiateils (1) ab. Die Führung dieser Bewegung erfolgt über einen Steg (24), der in eine gabelförmige Halterung (20) des Femurteils (5) eingeschoben ist und auf einem kreisscheibensektorförmigen Teil seines Umfangs eine Verzahnung (11) trägt. Diese Verzahnung greift in eine ebenfalls auf einem Kreisscheibensektor innerhalb der gabelförmigen Halterung (20) angeordneten Gegenverzahnung (16) ein.

Die Verzahnung (11, 16) erzwingt eine weitgehend geführte Abrollbewegung der beiden künstlichen Gelenkteile (1 und 5), wodurch die Annäherung des Bewegungsablaufs an den Verlauf der physiologischen Bewegung verbessert wird.

Fig. 2

EP 0 103 697 A1

0103697

P. 5750/Wg/IS

Gebrüder Sulzer, Aktiengesellschaft, Winterthur/Schweiz

Kniegelenkprothese

Die Erfindung betrifft eine Kniegelenkprothese aus einem Femurteil, der an einer gabelförmigen Halterung beiderseits einer Mittelebene kondylenähnlich geformte Tragflächen aufweist, und aus einem Tibiateil, der einen in die gabelförmige Halterung eingreifenden, scheibenförmigen Steg und beiderseits des Steges mit den Tragflächen zusammenwirkende Abrollflächen aufweist, wobei der Steg nach rückwärts durch den Sektor einer Kreisscheibe begrenzt ist.

Die Erfindung bezieht sich sowohl auf Endo-, d.h. in den menschlichen Körper implantierbare Gelenkprothesen, als auch auf Exoprothesen für künstliche Glieder und andere orthopädische Hilfsmittel, wie z.B. Schienen.

Eine Prothese der vorstehend genannten Art ist in Form einer Endoprothese aus der DE-OS 29 33 124 bekannt. Bei dieser Konstruktion, die - ebenso wie die vorliegende - für einen Einsatz bei fehlenden oder nicht mehr funktionstüchtigen Seiten- und Kreuzbändern bestimmt ist, ist mindestens während eines Teils einer Bewegung des Knies der kreisförmige Umfang des Stegs in einer entsprechenden, kreisförmigen Hohlzylinderfläche des Femurteils geführt, um einen physiologisch annähernd richtigen Bewegungsablauf zu gewährleisten. Der Kopf des Stegs und die Hohlzylinderfläche rollen bei diesem Bewegungsablauf gleitend aufeinander ab. Durch das reibende Gleiten der beiden Flächen aufeinander, das nur schwer zu kontrollieren und zu steuern ist, wird in die Bewegung eine kaum beeinflussbare Komponente "hineingetragen".

Aufgabe der Erfindung ist es, für eine weitere Annäherung an den physiologischen Bewegungsablauf ein unkontrolliertes Gleiten des einen Prothesenteiles auf dem anderen bei der Abrollbewegung möglichst zu vermeiden; gemäss der vorliegenden Erfindung wird diese Aufgabe dadurch gelöst. dass die gegen den Steg gerichtete Begrenzung der gabelförmigen Halterung des Femurteils in einem Bereich, der dem Kreisscheibensektor des Stegs gegenüberliegt, teilweise ebenfalls einen konvexen Kreisscheibensektor bildet, und dass beide Kreissektoren, über eine Verzahnung ineinandergreifend, aufeinander abwälzbar sind.

Die Ausbildung der beiden Kreisscheiben als Teilkreise einer Verzahnung beseitigt die Möglichkeit für ein unkontrolliertes Gleiten der beiden Führungsflächen aufeinander weitestgehend, wobei die ineinandergreifenden Zähne eine verbesserte Führung der Abrollbewegung der beiden Gelenkteile ermöglichen.

Um bei der neuen Gelenkprothese den Bewegungsablauf im Sinne einer Annäherung an die natürliche Bewegung zu optimieren, ist es zweckmässig, für die Teilkreise der Verzahnung und die Lage ihrer Mittelpunkte gewisse Grenzen einzuhalten:

So sollten - bei einer Auslegung, die vom Tibiateil als Basis ausgeht - die Teilkreisradien der Verzahnung die Beziehung $1,15 \leq f_T \leq 2$, insbesondere $1,38 \leq f_T \leq 1,44$, erfüllen, wobei das Verzahnungsverhältnis $f_T$ das Verhältnis der Summe der beiden Teilkreisradien zu dem Teilkreisradius des Tibiateils ist; mit dem Femurteil als Basis sollte das entsprechende Verzahnungsverhältnis $2 \leq f_T \leq 6$, insbesondere $3,3 \leq f_T \leq 3,6$, sein. Unabhängig davon, welcher der beiden Prothesenteile als Ausgangsbasis gewählt wird, ist es weiterhin zweckmässig, wenn der Winkel $\alpha$ zwischen einer tangentialen, horizontalen Ebene durch den Sattelpunkt der Abrollflächen des Tibiateils bzw. durch den unteren Scheitelpunkt der Tragflächen des Femurteils - bei in einer Sagittal-Ebene vertikal stehender Achse - und der Verbindungsgeraden der Mittelpunkte der beiden Teilkreise in Streckstellung der Prothese im Wertebereich $55^{\circ} \leq \alpha \leq 65^{\circ}$ liegt.

Unter Beachtung der vorstehenden Grenzen für die Verzahnungsverhältnisse lassen sich weiterhin Bereiche ermitteln, die sich für die Lage der Teilkreis-Mittelpunkte und die Grösse der Teilkreis-Radien als günstig erwiesen haben. So sollte - mit dem Tibiateil als Ausgangsbasis - der Mittelpunkt des Teilkreises des Tibiateils von der Achse des Tibiateils nach vorn einen horizontalen Abstand im Bereich $7 \leq A \leq 20$ mm haben und für den Teilkreisradius des Tibiateils gelten $25 \leq R_T \leq 37$ mm, wobei die Werte für den Abstand A und den Radius $R_T$ parallel zueinander und zur Grösse der Prothese wachsen sollten; für den vertikalen Abstand des Mittelpunkts von der horizontalen Sattelpunktebene nach unten sollte dabei eingehalten werden $-5 \leq B \leq 5$ mm. Nimmt man den Femurteil als Ausgangsbasis, so sollte der Mittelpunkt des Teilkreises des Femurteils von der Achse des Femurteils nach hinten einen horizontalen Abstand im Bereich $10 \leq C \leq 28$ mm haben und für den Teilkreisradius des Femurteils gelten $11 \leq R_F \leq 17$ mm; der vertikale Abstand des Mittelpunkts von der horizontalen Scheitelpunktebene sollte in diesem Fall $32 \leq D \leq 42$ mm betragen.

Durch die Festlegung der Teilkreisradien $R_T$ und $R_F$, ihrer Mittelpunkte $M_T$ und $M_F$ und des Winkels $\measuredangle$ ergibt sich in Verbindung mit der Form der Abrollflächen des Tibiateils konstruktiv die Form der Femurteil-Kondylen von ihrem erwähnten unteren Scheitelpunkt nach rückwärts, indem man die beiden Teilkreise aufeinander abrollen lässt; die Abrollfläche des Tibiateils bestimmt bei diesem Abrollen mit dem Punkt, der jeweils momentan den kürzesten Abstand zum Berührungspunkt der beiden aufeinander abrollenden Teilkreise hat, die die Kondylenform erzeugende Kurve.

Eine bevorzugte Ausführungsform der Verzahnung kann aus abwechselnd aufeinander folgenden halbzylinder- und halbkugelförmigen Abwälznoppen und -vertiefungen bestehen; die Bolzen sind dabei zweckmässigerweise aus Metall gefertigt, während die Vertiefungen in Kunststoffkörpern eingelassen sind, so dass beim Abrollen der Verzahnung direkte Kontakte zwischen relativ zueinander bewegten Metalloberflächen vermieden werden. Es ist jedoch auch möglich, eine andere der in der Technik bekannten Verzahnungen, z.B. eine Evolventen-Verzahnung, zu verwenden.

Um ein Ausklinken der Verzahnung im normalen Bewegungsbereich des Gelenkes zu verhindern, kann im Steg des Tibiateils ein Führungsbolzen vorgesehen sein, der in kulissenartige Führungen des Femurteils eingreift. Bolzen und Kulisse können dabei gleichzeitig ausserhalb des normalen Bewegungsbereichs eine Führung für das Ein- und Ausfädeln der beiden Gelenkteile bilden.

Als Materialien für die neue Prothese sind die für die Herstellung von Gelenkimplantaten bzw. für Gelenke künstlicher Glieder gebräuchlichen Materialien und Materialkombinationen geeignet.

Im folgenden wird die Erfindung anhand von Ausführungsbeispielen im Zusammenhang mit der Zeichnung näher erläutert; die Erfindung wird dabei im Zusammenhang mit Endoprothesen beschrieb

Fig. 1 zeigt in einer Ansicht auf eine Sagittalebene des Körpers an einer in Streckstellung schematisch dargestellten Gelenkprothese die Teilkreisradien sowie die Lage der Teilkreis-Mittelpunkte relativ zu den Achsen der beiden Gelenkteile und zur horizontalen Sattel- bzw. Scheitelpunktebene;

Fig. 2 ist - teilweise in einem Schnitt II-II von Fig. 3 - in einer Sagittalebene gesehen ein erstes Ausführungsbeispiel der Erfindung;

Fig. 3 gibt den Schnitt III-III von Fig. 2 wieder;

Fig. 4 ist eine Detailvariante der Fig. 3, während

Fig. 5 schliesslich eine weitere Ausführungsform der Erfindung darstellt.

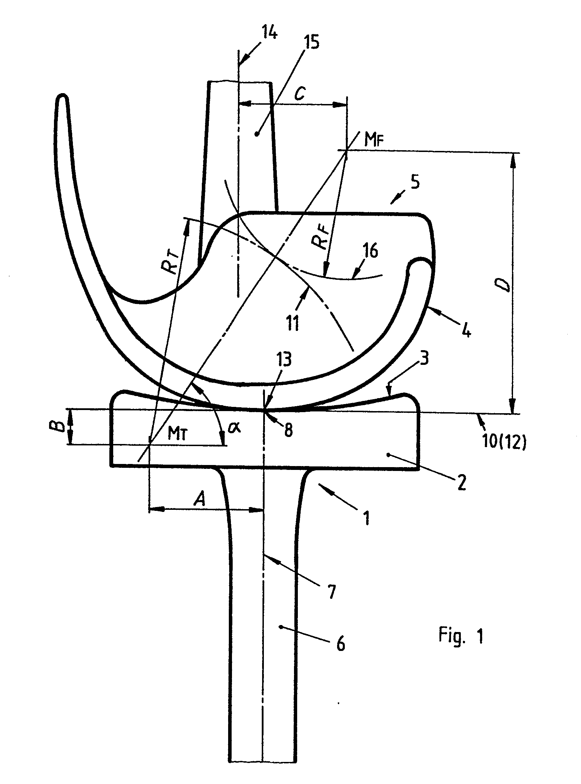

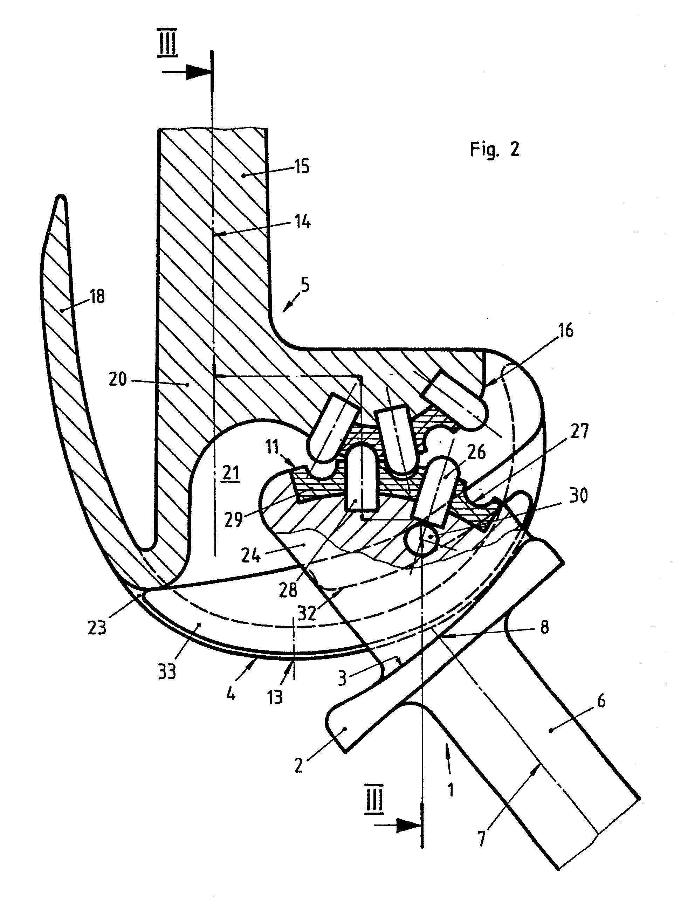

Der Tibiateil 1 (Fig. 1) der Prothese besteht im wesentlichen aus einer horizontalen Plattform 2, deren Oberfläche durch die kreisförmigen Abrollflächen 3 für die kondylenartigen Tragflächen 4 des Femurteils 5 gebildet wird. Für die Verankerung im Knochen ist an die Plattform 2 ein Verankerungsschaft 6 angesetzt; dessen Achse, die gleichzeitig die Achse des Tibiateils 1 ist, ist mit 7 bezeichnet. Bei der gezeigten Konstruktion schneidet sie die Abrollflächen 3 in deren Sattelpunkt 8. Durch diesen verläuft eine horizontale Bezugsebene 10, von der aus der vertikale Abstand B des Mittelpunkts $M_T$ für einen Teilkreis 11 einer Verzahnung (Fig. 2) des Tibiateils 1 festgelegt wird; der Teilkreisradius für die Verzahnung des Tibiateils ist mit $R_T$ bezeichnet.

In der in Fig. 1 gezeigten Streckstellung der Prothese fällt die Ebene 10 zusammen mit der horizontalen Ebene 12 durch den unteren Scheitelpunkt 13 der kondylenartig geformten Tragflächen 4 des Femurteils 5. Ebenso fällt in dieser Stellung der Scheitelpunkt 13 mit dem Sattelpunkt 8 des Tibiateils zusammen, um die direkte Uebertragung der Standbelastung von den Tragflächen 4 des Femurteils 5 auf die Abrollflächen 3 des Tibiateils 1 zu bewirken. Relativ zur Femurachse 14, die - in einer lateralen Ansicht, d.h. in einer Sagittalebene - gleichzeitig die Achse von dessen Schaft 15 ist, ist daher der Scheitelpunkt 13 etwas nach hinten, beispielsweise um 8 - 12 mm, versetzt. Die Lage des Punktes 13 bei vertikal stehender Achse des Femurteils bestimmt die horizontale Scheitelpunktebene 12, von der aus die vertikale Höhe D für den Mittelpunkt $M_F$ eines Femurteilskreises 16 gemessen wird, dessen Radius $R_F$ ist.

Die horizontalen Abstände A bzw. C der Mittelpunkte $M_T$ bzw. $M_F$ ergeben sich - wenn der Tibiateil 1 die Auslegebasis der Prothese ist - von der Achse 7 des Tibiateils aus nach vorn bzw. - wenn der Femurteil als Basis dient - von der Achse 14 des Femurteils 5 aus nach hinten. Sind die Lage des Mittelpunktes M des Teilkreises für den als Basis herangezogenen Gelenkteil festgelegt, der Teilkreisradius R dieses Teils und das Verzahnungsverhältnis f bestimmt, so hängen Teilkreisradius und Lage des Mittelpunktes des "abhängigen" Gelenkteils ausschliesslich vom Winkel $\measuredangle$ ab, den die Verbindungslinie zwischen den beiden Mittelpunkten $M_T$ und $M_F$ mit der horizontalen Sattelpunkt- 10 bzw. Scheitelpunktebene 12 in der Streckstellung des Gelenkes bildet; dieser Winkel wird innerhalb der angegebenen Grenzen bei der Auslegung der Prothese ebenfalls vorgewählt.

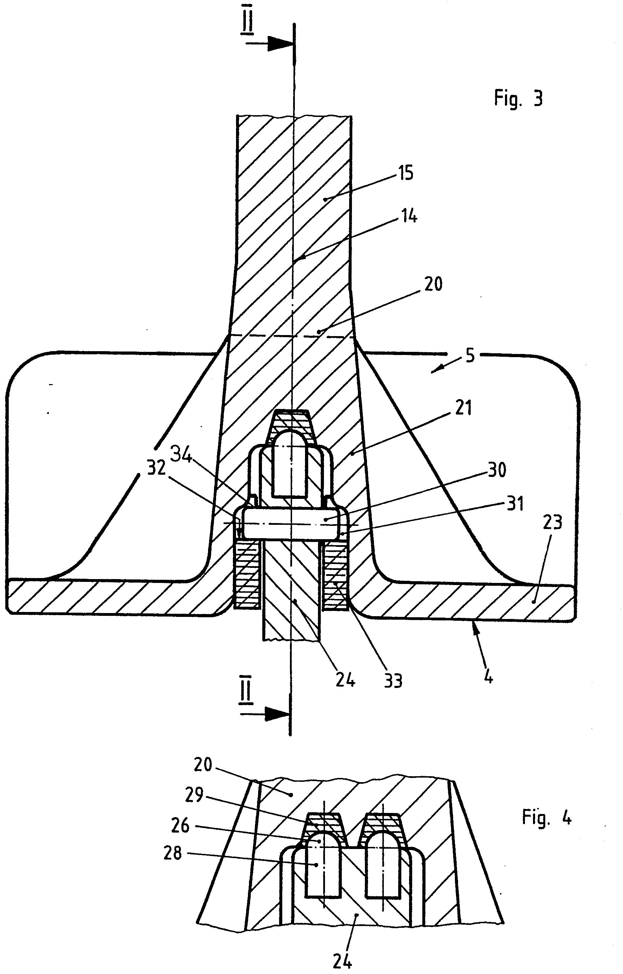

Wie aus Fig. 2 und 3 zu entnehmen ist, geht der Schaft 15 des Femurteils über in eine gabelförmige Halterung 20 mit nach hinten verlängerten Seitenwänden 21, an deren unteren Enden mit Tragflächen 4 versehene Schalen 23 angesetzt sind; der vordere Teil der Tragfläche 4, d.h. der Bereich, der vom Scheitelpunkt 13 in Fig. 2 nach links verläuft und in einer Auflage 18 für die nicht dargestellte Kniescheibe endet, stellt im Seitenriss eine aus Kreisbögen mit verschiedenen Radien und unterschiedlichen Mittelpunkten erzeugte Kurve dar; Form und Abmessungen dieser Kurve sind empirisch in etwa dem Verlauf der natürlichen Kondylenflächen nachgebildet. Abgesehen von der Abstützung der Kniescheibe hat dieser Teil für die Funktionsweise der Prothese keine Bedeutung.

Die Form für den hinteren Teil der kondylenartigen Tragflächen 4 ergibt sich, wie bereits beschrieben, durch den Weg der Abrollflächen 3 des Tibiateils 1 beim Abrollen der beiden Teilkreise 11 und 16 aufeinander; im Schnitt senkrecht zur sagittalen Richtung des Körpers sind die Tragflächen 4 - wie Fig. 3 erkennen lässt - eben ausgebildet.

Beim Tibiateil 1, der gegen unbeabsichtigte Drehungen im Schienbein gesichert ist, sind die dem Femur zugewandten Abrollflächen 3 in Anlehnung an die Oberfläche natürlicher Schienbeinknochen im Seitenriss als einfache Kreisbögen ausgebildet. Zwischen den beiden Abrollflächen 3 ist ein Steg 24 angeordnet, der im wesentlichen die Form eines Kreisscheiben-Quadranten besitzt. Erfindungsgemäss ist die Peripherie dieser Kreisscheibe als Zahnkranz ausgebildet; dieser besteht in dem gezeigten Beispiel aus abwechselnd aufeinanderfolgenden, halbkugelförmig vorstehenden Noppen 26 und entsprechenden Vertiefungen 27. Die Noppen 26 bilden dabei das freie Ende von metallischen Bolzen 28, die in den Steg 24 eingelassen und mit diesem fest verbunden sind, während die Vertiefungen 27 in zwischen dem Bolzen 28 ebenfalls im Steg 24 befestigte - z.B. eingeklebte - Kunststoffkörper 29 eingelassen sind. Weiterhin ist in einer Bohrung des Stegs 24 ein Führungsbolzen 30 vorgesehen, dessen Funktion später beschrieben wird.

Der zweite Zahnkranz der Verzahnung mit dem Teilkreis 16 befindet sich im Femurteil 5 an der "Decke" der nach hinten verlängerten, gabelförmigen Halterung 20; sie besteht aus den gleichen Noppen und Vertiefungen 26 bzw. 27 wie der Zahnkranz im Tibiateil 1.

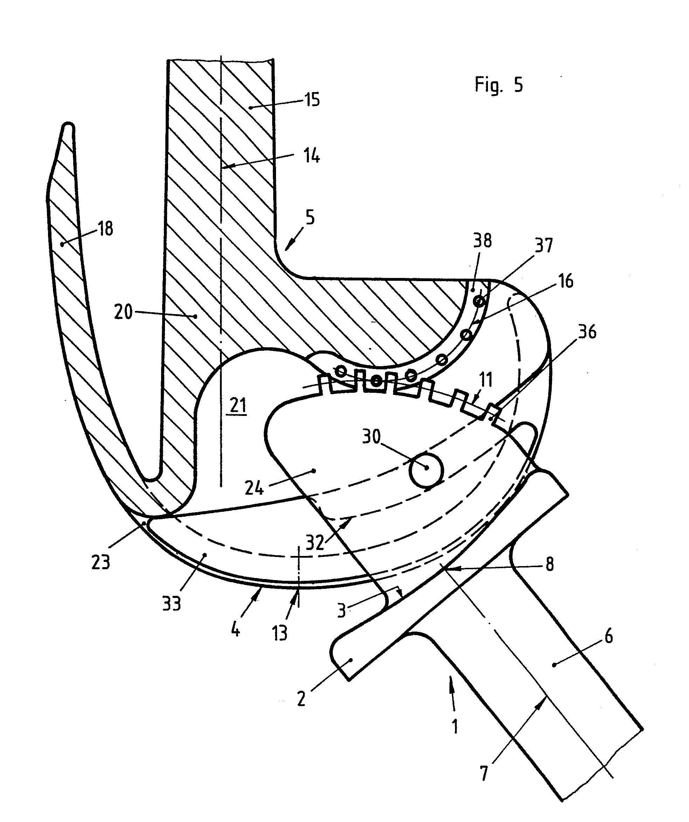

Die Zahnkränze können dabei einreihig - wie in Fig. 3 - oder zweireihig ausgeführt sein; eine Ausführungsform mit zwei Zahnreihen zeigt Fig. 4.

Wie aus Fig. 3 zu ersehen ist, ist die Gabel der Halterung 20 in ihrem unteren Teil erweitert; in den Erweiterungen 31 sind aus Kunststoff bestehende, mit Kulissen 32 versehene Führungselemente 33 angebracht. In diesen Kulissen 32 gleitet bei einer Bewegung im normalen Bewegungsbereich der Führungsbolzen 30 als Sicherung gegen ein ungewolltes Ausklinken der Verzahnung. Gleichzeitig ist die Kulissenführung 32 nach hinten derart verlängert, dass zwischen einem die Erweiterung 31 erzeugenden Absatz 34 der gabelförmigen Halterung und dem Führungselement 33 eine schlitzartige Führung entsteht, durch die das Ein- und Ausfädeln der beiden Prothesenteile 1 und 5 während der Implantationsoperation und das korrekte Eingreifen der beiden Zahnkränze ineinander erleichtert werden.

Eine konstruktiv besonders einfache Konstruktion einer Verzahnung zeigt Fig. 5. Der einen Kreissektor bildende Teil des Umfangs des Stegs 24 ist in diesem Fall mit einer Anzahl von im Abstand voneinander angeordneten, vorstehenden Bolzen 36 bestückt, während im Kreissektor der Halterung 20 an der "Decke" in entsprechenden Abständen in einem bogenförmigen Schlitz 38 verteilte, leitersprossenartige Holmen 37 vorgesehen sind, zwischen die die Bolzen 36 beim Abrollen der beiden Gelenkteile 1 und 5 eingreifen.

Form, Abmessungen und Lage der Trag- und Abrollflächen 4 bzw. 3, des Stegs 24 und der Teilkreisradien $R_T$ und $R_F$ sind so aufeinander abgestimmt, dass die statischen und dynamischen Kräfte, zumindest theoretisch, vollständig, in der Praxis jedoch lediglich weitgehend, von den kondylen- und tibiaartig ausgebildeten Trag- und Abrollflächen 4 bzw. 3 aufgenommen werden und die beiden Verzahnungen in Verbindung mit den genannten Trag- und Abrollflächen nur zur Führung der Bewegung des Knies dienen.

Femur- und Tibiateil 5 bzw. 1 bestehen vorteilhafterweise aus einem für Endoprothesen üblichen Metall oder einer Metall-Legierung; es ist jedoch auch möglich, sie - gegebenenfalls teilweise - aus Biokeramik, pyrolytischem Kohlenstoff oder einem anderen der für Endoprothesen bewährten Werkstoffe, z.B. aus Polyäthylen, bzw. Werkstoffkombinationen herzustellen. Um dabei soweit wie möglich ein Reiben

**0103697**

zwischen zwei relativ zueinander bewegten Metallteilen zu verhindern, sind die Abrollflächen 3 aus Kunststoff, z.B. aus Polyäthylen, als reibungsarme Gleitschicht hergestellt, was nicht ausdrücklich gezeigt ist. Dadurch wird darüberhinaus gleichzeitig eine gewisse Elastizität und Dämpfung zwischen beiden Prothesenteilen, vor allem beim Belasten, erreicht.

Bei Anwendung der Erfindung an künstlichen Gliedern werden die Verankerungsschäfte 6 und 15 durch Verbindungselemente ersetzt, mit denen Tibia- und Femurteil an künstlichen Unter- bzw. Oberschenkeln befestigt werden.

Patentansprüche

1. Kniegelenkprothese aus einem Femurteil, der an einer gabelförmigen Halterung beiderseits einer Mittelebene kondylenähnlich geformte Tragflächen aufweist, und aus einem Tibiateil, der einen in die gabelförmige Halterung eingreifenden, scheibenförmigen Steg und beiderseits des Stegs mit den Tragflächen zusammenwirkende Abrollflächen aufweist, wobei der Steg nach rückwärts durch den Sektor einer Kreisscheibe begrenzt ist, dadurch gekennzeichnet, dass die gegen den Steg (24) gerichtete Begrenzung der gabelförmigen Halterung (20) des Femurteils (5) in einem Bereich, der dem Kreisscheibensektor des Stegs (24) gegenüberliegt, teilweise ebenfalls einen konvexen Kreisscheibensektor bildet, und dass beide Kreissektoren, über eine Verzahnung (26, 27; 36, 37) ineinandergreifend, aufeinander abwälzbar sind.

2. Endoprothese nach Anspruch 1, dadurch gekennzeichnet, dass die Radien der Kreisscheibensektoren als Teilkreisradien der Verzahnung die Beziehung $1,15 \leq f_T \leq 2$ erfüllen, wobei das Verzahnungsverhältnis $f_T$ das Verhältnis der Summe der beiden Teilkreisradien ($R_T$, $R_F$) zu dem Teilkreisradius ($R_T$) des Tibiateils ist ($f_T = (R_T + R_F)/R_T$).

3. Endoprothese nach Anspruch 1, dadurch gekennzeichnet, dass die Teilkreisradien der Verzahnung die Beziehung $2 \leq f_F \leq 6$ erfüllen, wobei das Verzahnungsverhältnis $f_F$ das Verhältnis der Summe der beiden Teilkreisradien ($R_F$, $R_T$) zu dem Teilkreisradius ($R_F$) des Femurteils ist ($f_F = (R_T + R_F)/R_F$).

4. Endoprothese nach Anspruch 2 oder 3, dadurch gekennzeichnet, dass der Winkel $\alpha$ zwischen einer tangentialen, horizontalen Ebene (10) durch den Sattelpunkt (8) der Abrollflächen (3) des Tibiateils (1) bzw. - bei in einer Sagittal-Ebene vertikal stehender Achse (14) - durch den unteren Scheitelpunkt (13) der Tragflächen (4) des Femurteils (5) und der Verbindungsradien der Mittelpunkte $(M_r, M_F)$ der beiden Teilkreise (11, 16) in Streckstellung der Prothese im Wertebereich $55° \leqq \alpha \leqq 65°$ liegt.

5. Endoprothese nach Anspruch 2, dadurch gekennzeichnet, dass das Verhältnis $f_T$ einen Wert zwischen 1,38 und 1,44 hat.

6. Endoprothese nach Anspruch 3, dadurch gekennzeichnet, dass das Verhältnis $f_F$ einen Wert zwischen 3,3 und 3,6 hat.

7. Endoprothese nach Anspruch 2 und 4, dadurch gekennzeichnet, dass der Mittelpunkt $(M_T)$ des Teilkreises (11) des Tibiateils (1) von der Achse (7) des Tibiateils (1) nach vorn einen horizontalen Abstand (A) im Bereich $7 \leqq A \leqq 20$ mm hat, dass ferner für den Teilkreisradius $(R_T)$ des Tibiateils (1) gilt $25 \leqq R_T \leqq 37$ mm, wobei die Werte für den Abstand (A) und den Teilkreisradius $(R_T)$ parallel zueinander und zur Grösse der Prothese wachsen, und dass schliesslich der vertikale Abstand (B) des Mittelpunktes $(M_T)$ von der horizontalen Sattelpunktsebene (10) nach unten $-5 \leqq B \leqq 5$ mm beträgt.

8. Endoprothese nach Anspruch 3 und 5, dadurch gekennzeichnet, dass der Mittelpunkt $(M_F)$ des Teilkreises (16) des Femurteils (5) von der Achse (14) des Femurteils (5) nach hinten einen horizontalen Abstand (C) im Bereich

$10 \leqslant C \leqslant 28$ mm hat, dass ferner für den Teilkreisradius ($R_F$) des Femurteils (5) gilt $11 \leqslant R_F \leqslant 17$ mm, wobei die Werte für den Abstand (C) und den Teilkreisradius ($R_F$) parallel zueinander und zur Grösse der Prothese wachsen, und dass schliesslich der vertikale Abstand (D) des Mittelpunktes ($M_F$) von der horizontalen Scheitelpunktebene (12) $32 \leqslant D \leqslant 42$ mm beträgt.

9. Endoprothese nach Anspruch 1, dadurch gekennzeichnet, dass die Verzahnung aus abwechselnd aufeinanderfolgenden, halbzylinder- oder halbkugelförmigen Abwälznoppen (26) und Abwälzvertiefungen (27) besteht.

10. Endoprothese nach Anspruch 1, dadurch gekennzeichnet, dass der Steg (24) des Tibiateils (1) einen Führungsbolzen (30) trägt, der in kulissenartige Führungen (32) des Femurteils (5) eingreift.

Fig. 1

0103687

Fig. 2

0103697

Fig. 3

Fig. 4

Fig. 5

**Europäisches Patentamt**

# EUROPÄISCHER RECHERCHENBERICHT

EP 83 10 7023

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | GB-A-2 067 412 (NEW YORK SOCIETY FOR THE RELIEF OF THE RUPTURED AND CRIPPLED) * Seite 2, Zeilen 55-66; Abbildungen 3,7A-7F * | 1 | A 61 F 1/03 |
| A | ENGINEERING IN MEDICINE, Band 10, Nr. 2, April 1981, WHITSTABLE, KENT (GB), D. ELAD u.a.: "Synthesis of a knee joint endopr osthesis is based on pure rolling", Seiten 97-104. * Seiten 99-102; Abbildungen 11-14 * | 1-3,5-8 | |
| A | MEDIZINISCH-ORTHOPAEDISCHE TECHNIK, Band 101, Nr. 6, November-Dezember 1981, BAD-BUCHAU (DE), W. THOMAS: "Biomechanische Gesichtspunkte zur Ko nstruktion von Kniegelenkendoprothesen", Seiten 1 69-175. * Abbildungen 11,12a-c * | 1 | RECHERCHIERTE SACHGEBIETE (Int. Cl. ³) A 61 F |
| A | US-A-4 209 861 (P.S. WALKER u.a.) * Zusammenfassung; Abbildungen 1,9-13 * | 1 | |
| A | US-A-2 883 982 (F.E. RAINEY) * Abbildungen 1,2 * | 1 | |
| | --- -/- | | |

Der vorliegende Recherchenbericht wurde für alle Patentansprüche erstellt.

| Recherchenort DEN HAAG | Abschlußdatum der Recherche 30-11-1983 | Prüfer WOLF C.H.S. |
|---|---|---|

Europäisches
Patentamt

**EUROPÄISCHER RECHERCHENBERICHT**

EP 83 10 7023

| | EINSCHLÄGIGE DOKUMENTE | | Seite 2 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
| A | DE-B-2 549 819 (SANITÄTSHAUS SCHÜTT & GRUNDEI) * Spalte 4, Zeilen 1-17; Abbildung 4 * | 10 | |
| | --- | | |
| A,D | DE-A-2 933 124 (SULZER AG) * Abbildungen 1,2 * | 1 | |
| | ----- | | |
| | | | RECHERCHIERTE SACHGEBIETE (Int. Cl. ³) |

Der vorliegende Recherchenbericht wurde für alle Patentansprüche erstellt.

| Recherchenort | Abschlußdatum der Recherche | Prüfer |
|---|---|---|
| DEN HAAG | 30-11-1983 | WOLF C.H.S. |